# EUROPEAN PATENT APPLICATION

(11) **EP 1 561 421 A1**
(43) Date of publication of application: **10.08.2005**
(21) Application number: 03772723.7
(22) Date of filing: 12.11.2003
(51) Int. Cl.: A61B 5/15

(54) **LANCET AND NEEDLE INSERTION DEVICE**

(30) Priority: 15.11.2002 JP 2002331603
(71) Applicant: ARKRAY, Inc., Kyoto-shi, Kyoto 601-8045 (JP)
(72) Inventor: SAKATA, Tetsuya, c/o ARKRAY, Inc., Kyoto-shi, Kyoto 601-8045 (JP)
(74) Representative: Pluckrose, Anthony William
(86) International application number: PCT/JP2003/014398
(87) International publication number: WO 2004/045409

(57) **Abstract**

The present invention relates to a lancet (A1) in which a first member (2) including a lancing element (1) and a second member (3) for accommodating the tip (1a) of the lancing element (1) are arranged movably relative to each other. In the lancet (A1), when a load greater than a predetermined value is applied in a direction to make the first and the second members (2, 3) approach each other, the tip (1a) of the lancing element (1) is capable of projecting from the second member (3) by relative movement between the first member (2) and the second member (3) in a direction to approach each other. The tip (1a) of the lancing element (1) can be accommodated in the second member (3) so as not to project from the second member (3) by relative movement between the first member(2) and the second member (3) in a direction to separate from each other.

## Description

### TECHNICAL FIELD

The present invention relates to a technique for drawing body fluid from the skin to measure e.g., the concentration of a test substance contained in the body fluid.

### BACKGROUND ART

Referring to Fig. 18 of the present application, an example of a conventional lancet (see e.g., JP-A-H08-597) comprises a lancet body 100 provided with a needle 101 and a screw portion 104, and a cap 110 is attached to the body in a manner such that it can be moved in a N1-N2 direction shown in the figures.

The cap 110 is made hollow to accommodate the needle tip 101a of the needle 101. The cap 110 includes a cover member 111 and a needle tip protection head 113 formed integral with the cover member 111. Between the cover member 111 and the needle tip protection head 113 is provided a constricted portion 114, so that the needle tip protection head 113 can be separated from the cover member 111 to expose the needle tip 101a of the needle 101. The cover member 111 has an inner surface formed with a screw engagement projection 112 engaging with an engagement portion 105 of the lancet body 100. Thus, upon rotation of the cover member 111 relative to the lancet body 100, the cover member 111 advances or retreats relative to the lancet body 100 in the N1-N2 direction, whereby the protrusion of the needle tip 101a can be adjusted.

In use, the lancet is mounted to a lancing apparatus (not shown), and the needle tip 101a is caused to project from the cover member 111. Specifically, the needle tip 101a is caused to project when the user holds the protective head 113 with fingers and twists it to cut the needle tip protection head 113 at the constricted portion 114. The user manually rotates the cover member 111 to adjust the amount of projection of the needle tip from the cover member 111, as required. After the lancing procedure is over, the needle tip 101a is concealed inside the cover member 111 for hygiene and safety. This operation is performed manually by the user by rotating the cover member 111 to move the cover member 111 in the N2 direction, i.e., away from the lancet body 100.

With the above-described lancet, however, the user uses his or her hand for handling the cover member 111 for adjusting the needle tip protrusion and concealing the needle tip 101a after the lancing operation, and this handling need be performed near the needle tip 101a when the tip 101a is exposed. Therefore, it is not safe in a sense that there is a considerable possibility that the user is hurt by the needle tip. In addition, it is troublesome for the user to handle the cover member 111. Also, the twisting to cut the needle tip protective head 113 need be performed near the needle tip 101a, which can be dangerous. Further, the number of parts to be discarded increases.

### DISCLOSURE OF THE INVENTION

An object of the present invention is to enhance the safety and convenience of the user in extracting body fluid.

According to a first aspect of the present invention, there is provided a lancet to be attached to a lancing apparatus for moving a lancing element in a lancing direction from a standby position toward a lance position. The lancet comprises a first member including a lancing element and a second member for accommodating a tip of the lancing element, wherein the first member and the second member are movable relative to each other. When a load greater than a predetermined value is applied in a direction to cause the first member and the second member approach each other, the first member is brought closer to the second member so that the tip of the lancing element is capable of projecting from the lancing element. On the other hand, when the first member is brought away from the second member, the tip of the lancing element is accommodated in the second member without projecting from the second member.

Preferably, the lancet according of the present invention further comprises a fixer for fixing the second member to the first member when the second member accommodates the lancing element.

For example, the fixer comprises a pair of projections which project at the first member in a direction crossing the lancing direction and which are spaced from each other in the lancing direction. An engagement portion is provided at the second member to be held between the paired projections.

For example, the pair of projections comprises a first projection and a second projection which is closer to the lance position than the first projection and which projects more than the first projection. In this case, when the second member moves relative to the first member in the lancing direction, the second projection engages with the engagement portion of the second member to serve as a stopper for controlling the movement of the second member.

For example, at least either of the pair of projections and the engagement portion is annular.

For example, the first member includes a hole for accommodating an end of the second member and allowing movement of the second member. The paired projections are formed at an inner surface of the hole. In this case, the hole has a bottom surface serving as a stopper for controlling the movement of the second member by engaging with the engagement portion of the second member when the second member moves relative to the first member in a direction opposite to the lancing direction.

For example, the first member includes an additional hole communicating with the above-mentioned hole and extending in the lancing direction. Appropriate force is applicable to the second member in the lancing direction via the additional hole.

Preferably, the second member accommodates the tip of the lancing element in a hermetically sealed state and includes a portion to be penetrated by the tip of the lancing element when the first and the second members are moved to approach each other.

For example, the portion to be penetrated is integrally formed with the second member. In this case, the portion to be penetrated is provided at a position retreating, in a direction opposite to the lancing direction, from an end surface of the second member on the lancing direction side. Alternatively, the portion to be penetrated may be provided at the end surface. The portion to be penetrated may be provided by a sheet member attached.

According to a second aspect of the present invention, there is provided a lancing apparatus which is used by mounting a lancet and which moves the lancet in a lancing direction from a standby position toward a lance position. The lancet comprises a first member including a lancing element and a second member for accommodating a tip of the lancing element, where the first member and the second member are movable relative to each other. When a load greater than a predetermined value is applied in a direction to cause the first member and the second member to approach each other, the first member is brought closer to the second member so that the tip of the lancing element is capable of projecting from the lancing element, whereas when the first member is brought away from the second member, the tip of the lancing element is accommodated in the second member without projecting from the second member. The lancing apparatus comprises a lancet holder for holding the lancet, the lancet holder being movable in the lancing direction. The lancing apparatus also comprises a mover which is movable relative to the lancet holder for moving the second member relative to the first member in the lancing direction, and for causing the tip of the lancing element projecting from the second member to be brought into the second member to be accommodated in the second member.

Preferably, the mover moves in the lancing direction to engage with the second member and moves the second member relative to the first member in the lancing direction, and thereafter pushes the lancet out of the lancet holder.

According to a third aspect of the present invention, there is provided a lancet to be attached to a lancing apparatus for moving a lancing element from a standby position toward a lance position. The lancet comprises a first member including a lancing element and a second member for accommodating a tip of the lancing element. The position of the lancing element relative to the second member is adjustable by a rotational force applied from the lancing apparatus.

The first member or the second member is rotated by the rotational force applied from the lancing apparatus, for example. Preferably, in this case, the first member or the second member is provided with an engagement portion to which the rotational force from the lancing apparatus is applied.

The lancet of the present invention may further comprise a third member which is movable relative to the first member in the movement direction of the lancing element and which is rotatable relative to the second member. The adjustment of the position of the lancing element relative to the second member may be performed by rotating the third member by the rotational force applied from the lancing apparatus. Preferably, in this case, the third member is provided with an engagement portion to which the rotational force from the lancing apparatus is applied.

Preferably, the lancing element is accommodated in a hermetically sealed state. In such a case, by sterilizing the lancing element by e.g. γ-ray irradiation with the airtightness maintained, the sterilized state of the lancing element can be maintained until the lancing element is exposed for lancing.

According to a fourth aspect of the present invention, there is provided a lancing apparatus to which a lancet is attached and which is designed to move the lancet from a standby position toward a lance position. The lancet comprises a first member including a lancing element and a second member for accommodating a tip of the lancing element, wherein the position of the lancing element relative to the second member is adjustable by application of a rotational force. The lancing apparatus also comprises a rotator for applying, to the lancet, the rotational force for adjusting the relative position of the lancing element.

Preferably, the lancet accommodates the lancing element in a hermetically sealed state. Before lancing is performed, the rotator moves the lancing element in the lancing direction to break the hermetically sealed state, and causes the lancing element to project from the second member.

### BRIEF DESCRIPTION OF THE DRAWINGS

Fig. 1 is a sectional perspective view of a lancet according to a first embodiment of the present invention.
Fig. 2 is a sectional view taken along lines II-II in Fig. 1.
Fig. 3A is a sectional view taken along lines III-III in Fig. 1, Fig. 3B is a sectional view showing the state in which the lancing needle projects, and Fig. 3C is a sectional view showing the state in which the lancing needle is accommodated again.
Fig. 4 is a sectional view showing a principal portion in a state in which the lancet is mounted to a lancing apparatus.
Fig. 5 is a sectional view of a principal portion for describing the lancing operation.
Fig. 6 is a sectional view of a principal portion for describing the lancing operation.
Fig. 7 is a sectional view of a principal portion for describing the operation for accommodating the lancing needle again.
Fig. 8 is a sectional view of a principal portion for describing the operation for detaching the lancet.
Figs. 9A-9C are sectional perspective views showing other examples of smaller cylindrical portion of a cap of a lancet.
Fig. 10 is a sectional view, partially cut away, showing a lancet according to a second embodiment of the present invention.
Fig. 11A is a sectional view taken along lines XI-XI in Fig. 10, Fig. 11B is a sectional view showing the state in which the lancing needle projects, and Fig. 11C is a sectional view showing the state in which the lancing needle is accommodated again.
Fig. 12 is a sectional view showing a principal portion in a state in which the lancet is mounted to a lancing apparatus.
Fig. 13 is a sectional view of a principal portion for describing the lancing operation.
Fig. 14 is a sectional view of a principal portion for describing the lancing operation.
Fig. 15 is a sectional view of a principal portion for describing the operation for accommodating the lancing needle again.
Fig. 16 is a sectional view for describing another example of lancet.
Fig. 17 is a sectional view taken along lines XVII-XVII in Fig. 16.

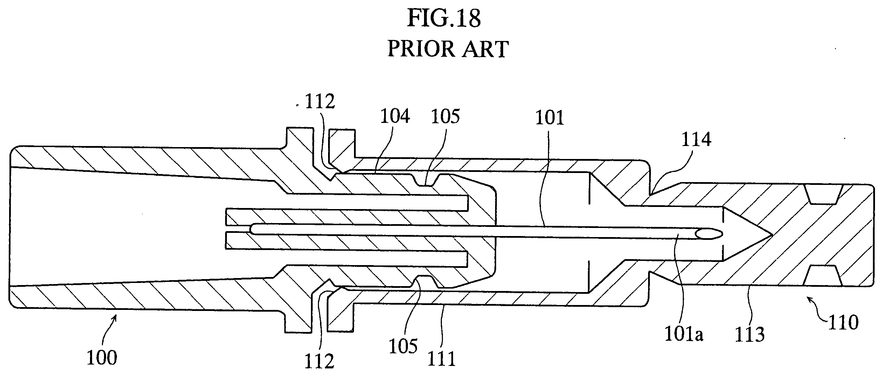

Fig. 18 is a sectional view for describing a prior art lancet.

### BEST MODE FOR CARRYING OUT THE INVENTION

Preferred embodiments of the present invention will be described below in detail with reference to the accompanying drawings.

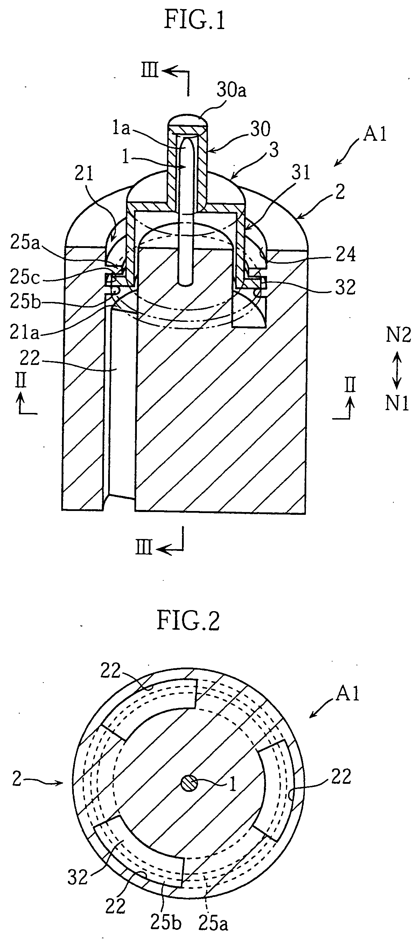

First, a first embodiment of the present invention will be described with reference to Figs. 1 through 8.

As shown in Figs. 1 through 3A, the lancet A1 includes a lancet body 2 having a lancing needle 1, and a cap 3 for accommodating the lancing needle 1.

The lancet body 2 is formed with an annular hole 21 and three through-holes 22.

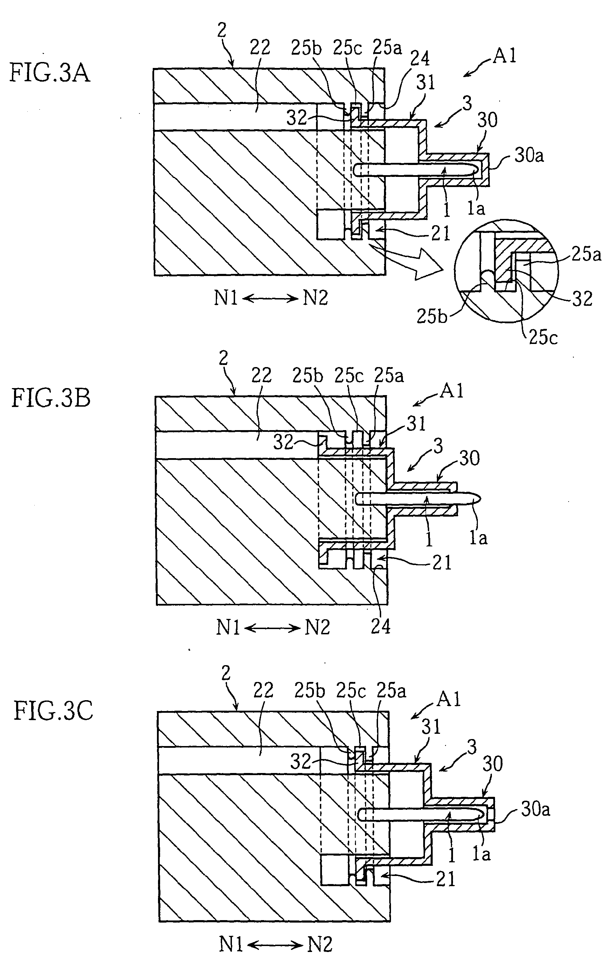

As better shown in Figs. 3A and 3B, the annular hole 21 serves to receive an end of the cap 3 while allowing the movement of the cap 3 relative to the lancet body 2. As shown in Figs. 1 through 3A, the hole 21 has an inner surface 24 formed with a first and a second annular projections 25a and 25b projecting radially inward. The amount of projection of the first annular projection 25a is greater than that of the second annular projection 25b. When the cap 3 is moved relative to the lancet body 2 in the N2 direction in the figure, the first annular projection 25a comes into engagement with a flange 32 of the cap 3, which will be described later, to stop the movement of the cap 3 in the N2 direction. The first and the second annular projection 25a and 25b are spaced from each other by a predetermined distance in the direction indicated by the arrows N1-N2, thereby producing a recess 25c between the projections 25a and 25b. The recess 25c engages with the flange 32 of the cap 3, which will be described later, to fix the cap 3 to the lancet body 2.

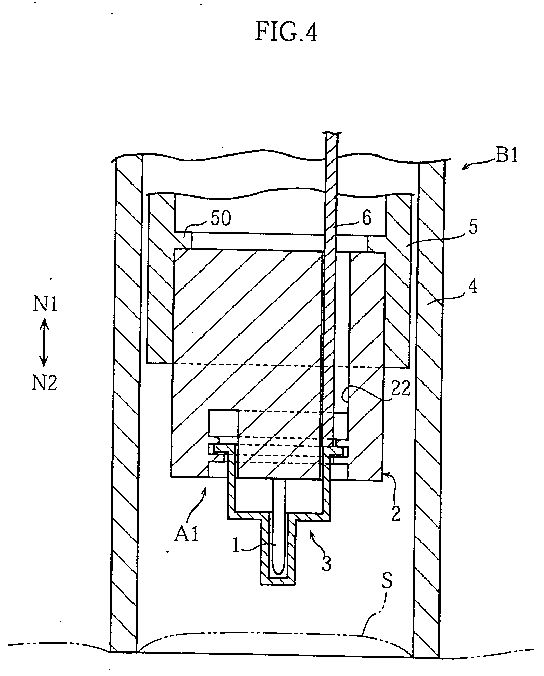

Each of the through-holes 22 serves to allow the movement of a pusher 6 (See Fig. 4) of a lancing apparatus B1, which will be described later. Each through-hole 22 communicates with the annular hole 21 at a bottom surface 21 of the annular hole 21 and extends in the N1-N2 direction. The configuration and number of the through-holes 22 are not limited to those illustrated in the figures.

The cap 3 serves to accommodate the tip 1a of the lancing needle 1 of the lancet body 2 in a hermetically sealed state. The cap 3 includes a smaller cylindrical portion 30, a larger cylindrical portion 31 and a flange 32.

The smaller cylindrical portion 30 includes a closed front end 30a and an open base end. The front end 30a of the smaller cylindrical portion 30 is penetrated by the lancing needle 1 when the lancet body 2 moves in the N2 direction relative to the cap 3. The front end 30a of the smaller cylindrical portion 30 and the other part of the cylindrical portion 30 are formed integral, where the front end is smaller in thickness than the other part so that it can be easily penetrated by the lancing needle 1.

The larger cylindrical portion 31 has a larger diameter than the smaller cylindrical portion 30, and is formed integral with the smaller cylindrical portion 30 to provide a continuous hole.

The flange 32 is used for fixing the cap 3 to the lancet body 2. Specifically, the flange 32 projects radially outward at an end of the larger cylindrical portion 31, and the cap 3 is fixed to the lancet body 2 by fitting the flange 32 into the recess 25c of the lancet body 2. Before the lancing operation, the flange 32 is held in the recess 25c, and in this state, the airtightness inside the cap 3 is maintained. Therefore, when the lancing needle tip 1a is sterilized by e.g. γ-ray irradiation with the airtightness maintained, the sterilized state of the lancing needle 1 can be maintained until the lancing needle 1 is exposed for lancing. The flange 32 also serves to control the range of movement of the lancing needle 1. Specifically, the flange 32 engages with the bottom surface 21a of the annular hole 21 when the cap 3 moves relative to the lancet body 2 in the N1 direction, while it engages with the first annular projection 25a when the cap 3 moves relative to the lancet body 2 in the N2 direction.

In the lancet A1, the amount of projection of the second annular projection 25b is smaller than that of the first annular projection 25a. Therefore, as shown in Fig. 3B, when a load greater than a predetermined value is applied to the cap 3 in the N1 direction, the flange 32 disengages from the recess 25c. Thus, the cap 3 can move relative to the lancet body 2 in the N1 direction (in the direction in which the cap 3 approaches the lancet body 2). When the cap 3 is moved relative to the lancet body 2 by a distance greater than a predetermined value in the N1 direction, the tip 1a of the lancing needle 1 breaks through the front end 30a of the cap 3, whereby the lancing needle 1 projects from the cap 3. The movement of the cap 3 relative to the lancet body 2 is stopped by the engagement of the flange 32 of the cap 3 with the bottom surface 21a of the annular hole 21 of the lancet body 2.

In the state shown in Fig. 3B, when a load greater than a threshold is applied to the cap 3 in the N2 direction, the flange 32 moves over the second annular projection 25b and engages with the first annular projection 25a, whereby the movement of the cap 3 is stopped. In this way, as shown in Fig. 3C, the flange 32 is again held in the recess 25c, thereby fixing the cap 3 to the lancet body 2. The lancing needle 1, once caused to project from the cap 3, is brought back into the cap 3.

Next, the usage of the lancet A1 and the lancing apparatus B1 to which the lancet A1 is to be applied will be described with reference to Figs. 4 through 8.

As shown in Fig. 4, the lancing apparatus B1 includes a housing 4, a lancet holder 5 and three pushers 6.

The lancet holder 5, holding the lancet A1, moves the lancet A1 in the N2 direction. The lancet holder 5 is accommodated in the housing 4 and is movable in the N1-N2 direction. The lancet holder 5 is provided with a stopper 50. When the lancet A1 is attached to the lancet holder 5, the lancet A1 is properly positioned by the stopper 50. For moving the lancet holder 5 in the N2 direction, use may be made of a known mechanism such as a latch mechanism, an electromagnetic mechanism, or a pneumatic mechanism.

The three pushers 6 serve to move the cap 3 of the lancet A1 in the N2 direction. Each of the pushers 6 is accommodated in the housing 4 and is movable in the N1-N2 direction independently from the lancet holder 5.

To extract body fluid using the lancet A1 and the lancing apparatus B1, the lancet A1 is first mounted to the lancing apparatus B1. Specifically, the lancet A1 is mounted by fitting the lancet body 2 to the lancet holder 5 by positioning the pushers 6 relative to the through-holes 22 and inserting the pushers into the through-holes.

To facilitate the insertion of the pushers 6 into the through-holes 22, the lancet A1 and the lancet holder 5 may be provided with a positioning mark, for example.

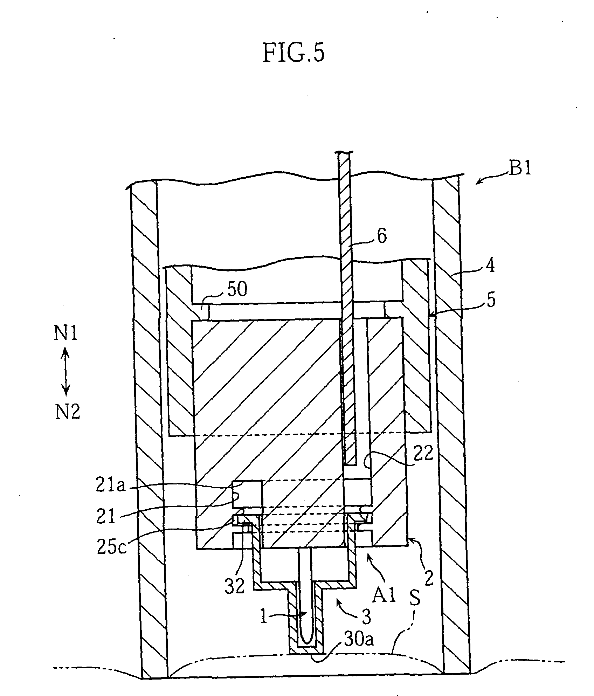

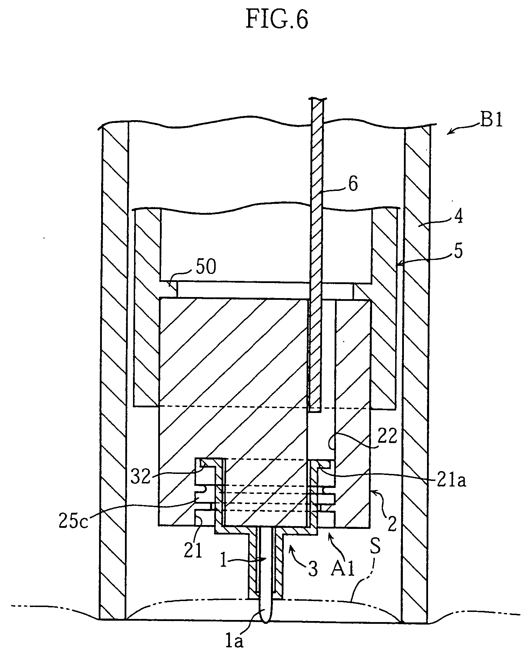

Subsequently, as shown in Fig. 5, after the front end of the housing 4 is brought into contact with the skin S, the lancet holder 5 is moved in the N2 direction. Thus, the lancet A1 moves together with the lancet holder 5 in the N2 direction, i.e., toward the skin S. When the front end 30a of the lancet A1 comes into contact with the skin S, a force in the N1 direction is exerted to the cap 3. As a result, as shown in Fig. 6, the flange 32 of the cap 3 disengages from the recess 25c of the lancet body 2 so that the cap 3 moves relative to the lancet body 2 in the direction to approach the lancet body. This movement continues until the flange 32 engages with the bottom surface 21a of the annular hole 21 of the lancet body 2. Thus, the lancing needle 1 projects from the front end 30a of the cap 3 to lance the skin S. As a result, blood comes out from the skin S.

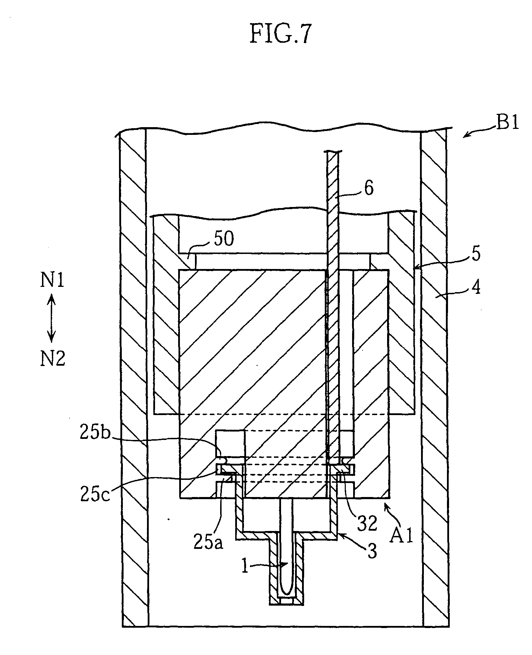

After the extraction of the body fluid is over, the lancet A1 will be detached from the lancing apparatus B1. In the present embodiment, as shown in Fig. 7, first the lancing needle 1 is retreated into the cap 3, and then the lancet A1 is detached. The retreat of the lancing needle 1 is performed by moving the pushers 6 relative to the lancet holder 5 in the N2 direction. More specifically, when the pushers 6 are moved in the N2 direction, the front ends of the pushers 6 push the flange 32 of the cap 3. As a result, the cap 3 moves away from the lancet body 2, i.e., moves in the N2 direction. In this movement, the flange 32 rides over the second annular projection 25b and engages with the first annular projection 25a, which prevents further movement of the cap relative to the lancet body 2. Thus, the flange 32 is again held into the recess 25c, and the cap 3 returns to its original position. In this way, the lancing needle 1 is accommodated again in the cap 3.

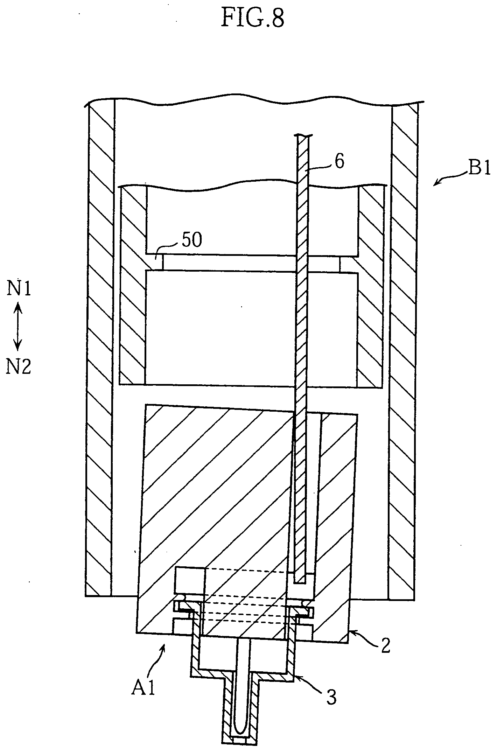

In the above-described state, the movement of the cap 3 relative to the lancet body 2 is prevented. Therefore, when the pushers 6 are moved further in the N2 direction, the lancet body 2 moves together with the cap 3 in the N2 direction. As a result, as shown in Fig. 8, the lancet A1 is pushed out of the lancet holder 5. Thus, in this embodiment, the returning of the lancing needle 1 into the cap 3 and the detachment of the lancet A1 from the lancet holder 5 can be performed as successive operations just by moving the pushers 6 in the N2 direction.

In the lancing apparatus B1, the pushers 6 may be moved manually by the operation of a lever or automatically by using a driver which utilizes a motor, for example.

In this embodiment, the user can cause the lancing needle 1 to project from the lancet A1 safely without touching the lancet A1 and without performing a complicated operation.

Further, in the above embodiment, the detachment of the lancet A1 after the lancing operation can be performed with the lancing needle 1 kept in the cap 3, and the returning of the lancing needle 1 into the cap 3 and the detachment of the lancet A1 from the lancet holder 5 can be performed by the user without touching the lancet A1. Therefore, the detachment and disposal of the lancet can be performed hygienically and safely.

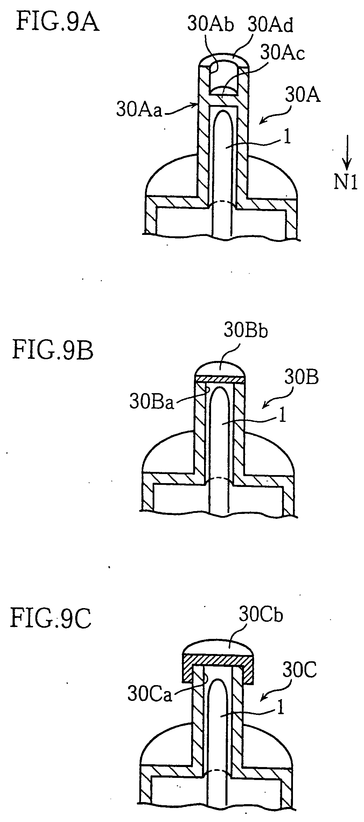

Although the cap including a smaller cylindrical portion and a larger cylindrical is exemplarily described in this embodiment, the configuration of the cap is not limited to that described above. The structure for holding the cap by the lancet body and returning the lancing needle into the cap are not limited to those described above. For example, the smaller cylindrical portion of the cap may have such a configuration as shown in Figs. 9A-9C.

Fig. 9A shows a smaller cylindrical portion 30A which has a front end 30Aa formed with a recess 30Ab opening toward the front. In the smaller cylindrical portion 30A, the portion to be penetrated by the lancing needle 1 (i.e., the bottom 30Ac of the recess 30Ab) is provided at a position retreating from the end surface 30Ad of the smaller cylindrical portion 30A in the N1 direction.

With such a structure, the portion to be penetrated (the bottom 30Ac of the recess 30Ab) does not come into direct close contact with the skin. Therefore, as compared with the case in which the portion to be penetrated (bottom 30Ac) comes into close contact with the skin, only a smaller load is necessary for causing the lancing needle 1 to project from the smaller cylindrical portion 30A.

Fig. 9B shows a smaller cylindrical portion 30B having a front end formed with an opening 30Ba which is closed by a seal 30Bb. The seal 30Bb is provided by bonding a film, which is made of metal or resin, by utilizing thermal energy, ultrasonic energy or an adhesive.

With such a structure, the thickness of the portion to be penetrated (seal portion 30Bb) can be made relatively small. Therefore, similarly to the foregoing case, only a smaller load is necessary for causing the lancing needle 1 to project from the smaller cylindrical portion 30B.

Fig. 9C shows a smaller cylindrical portion 30C having a front end formed with an opening 30Ca which is closed by a cap 30Cb.

With such a structure, the lancing can be performed with the cap 30Cb removed. Therefore, in lancing, the perforation of the smaller cylindrical portion 30C by the lancing needle 1 need not be performed.

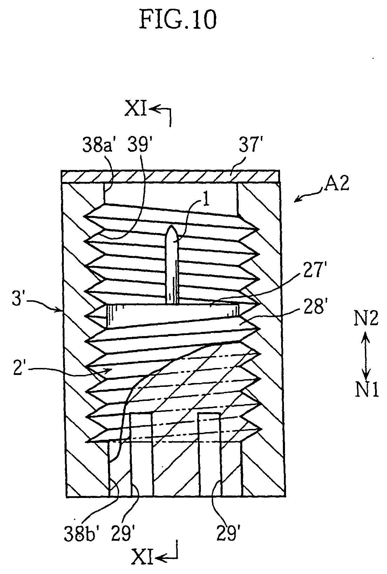

Next, a second embodiment of the present invention will be described with reference to Figs. 10 through 15. In these figures, the elements which are identical or similar to those of the lancet A1 and the lancing apparatus B1 of the first embodiment are designated by the same reference signs as those used for the first embodiment, and detailed description thereof is omitted.

As shown in Fig. 10, the lancet A2 includes a lancet body 2' having a lancing needle 1, and a cylindrical portion 3' for accommodating the lancing needle 1.

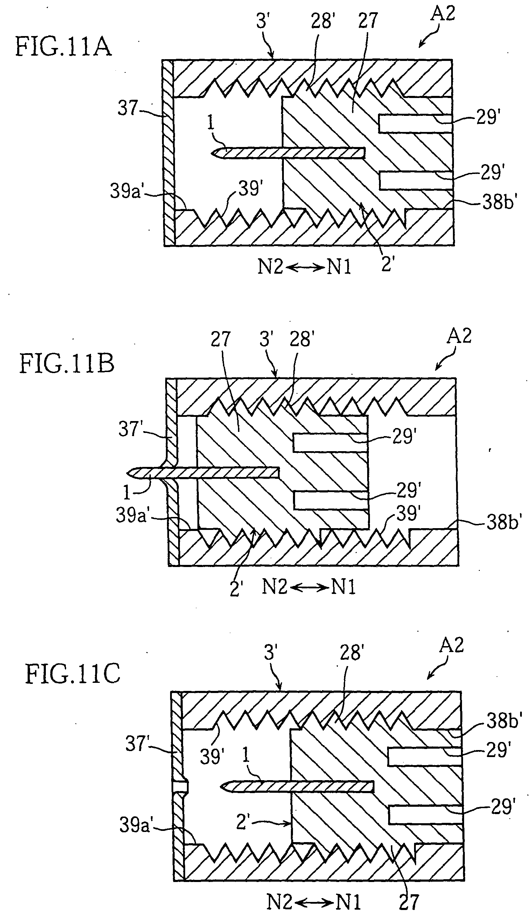

As shown in Figs. 10 and 11A, the cylindrical portion 3' has opposite ends formed with openings 38' and 38b'. The opening 38a' is closed by a seal member 37'. However, only the opening 38b' may be provided as the opening of the cylindrical portion 3'. The cylindrical portion 3' has an inner surface formed with a female thread 39'.

The lancet body 2' includes a holder portion 27' holding the lancing needle 1. The holder portion 27' has an outer circumferential surface formed with a male thread 28' for engagement with the female thread 39' of the cylindrical portion 3' . Therefore, when the lancet body 2' is rotated relative to the cylindrical portion 3', the holder portion 27' advances and retreats relative to the lancet body 2' in the direction indicated by the arrows N1, N2 in the figure. As shown in Fig. 11B, in lancing, the holder portion 27' is moved relative to the cylindrical portion 3' in the N2 direction, whereby the lancing needle 1 projects from the cylindrical portion 3'. After the lancing, as shown in Fig. 11C, the holder portion 27' is moved relative to the cylindrical portion 3' in the N1 direction, whereby the lancing needle 1 can be accommodated into the cylindrical portion 3'.

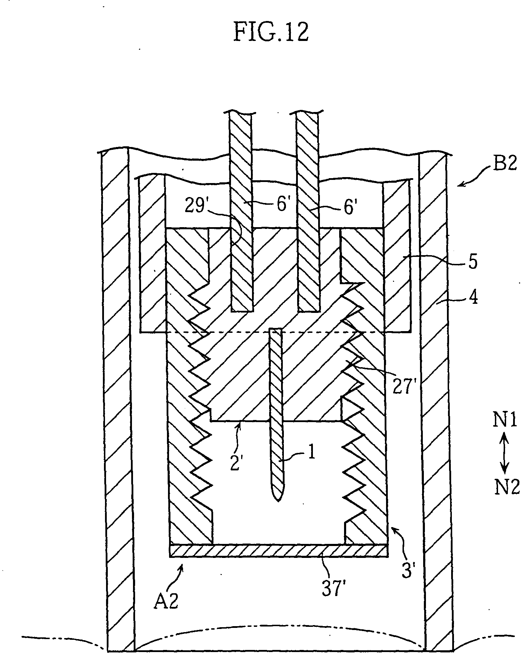

The lancet body 2' is formed with a pair of recesses 29'. The recesses 29' are provided for fitting a rotational member 6' (See Fig. 12) of a lancing apparatus B2, which will be described later. However, the position and configuration of the recesses 29' are not limited to those illustrated in the figures.

Next, the usage of the lancet A2 and the lancing apparatus B2 to which the lancet A2 is to be applied will be described with reference to Figs. 12 through 15.

As shown in Fig. 12, the lancing apparatus B2 includes a housing 4, a lancet holder 5 accommodated in the housing 4 and serving to hold the lancet A2, and a rotational member 6' .

The rotational member 6' serves to rotate the lancet body 2' of the lancet A2 in the circumferential direction of the housing. The rotational member 6', which is accommodated in the housing 4, is movable in the N1-N2 direction independently from the lancet holder 5 and rotatable in the circumferential direction of the housing 4. The rotational member 6' may be rotated manually by the operation of a lever or automatically by using a driver which utilizes a motor, for example.

To take body fluid, the lancet A2 is first mounted to the lancing apparatus B2. Specifically, the lancet A2 is mounted by fitting the cylindrical portion 3' of the lancet A2 to the lancet holder 5 by positioning the rotational member 6' relative to the recesses 29' of the lancet body A2 and inserting the rotational member into the recesses.

To facilitate the insertion of the rotational member 6' into the recesses 29', the lancet A2 and the lancet holder 5 may be formed with positioning marks.

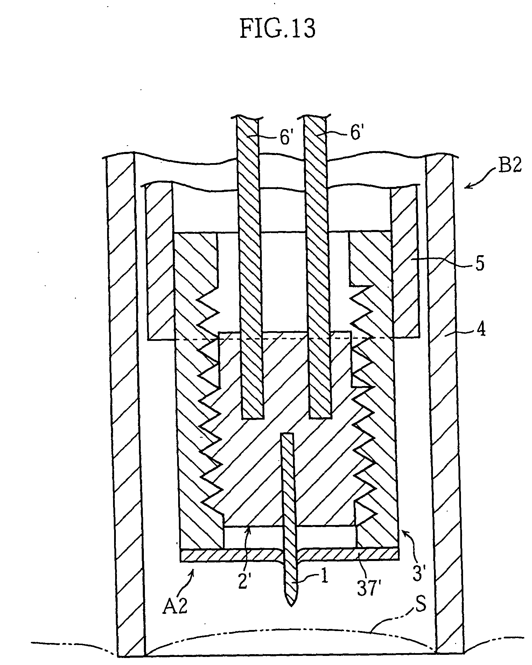

Subsequently, as shown in Fig. 13, with the front end of the housing 4 kept in contact with the skin S, the lancing needle 1 is caused to project from the cylindrical portion 3' through the seal member 37' . Specifically, the lancing needle 1 is caused to project by rotating the lancet body 2' relative to the cylindrical portion 3' by using the rotational member 6' to move the lancet body 2' in the N2 direction. The projecting operation of the lancing needle 1 may be performed before the housing 4 is brought into contact with the skin S.

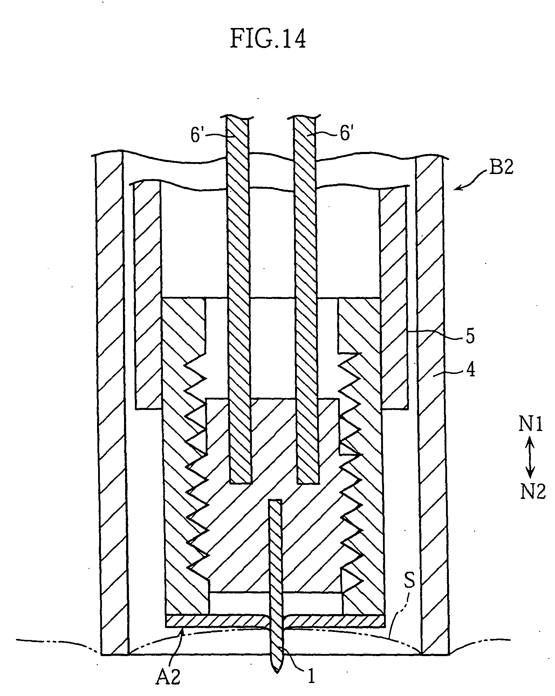

Subsequently, as shown in Fig. 14, the lancet holder 5 is moved in the N2 direction to lance the skin S by the lancing needle 1, whereby blood comes out from the skin S.

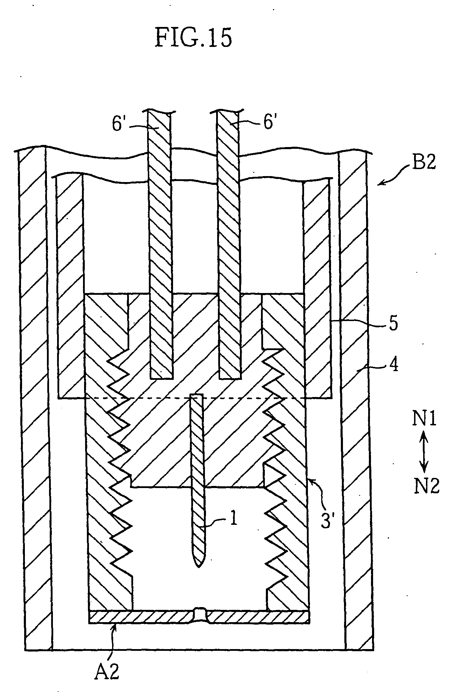

After the taking of the body fluid is completed, the lancet A2 is detached from the lancing apparatus B2. In this embodiment, before the detachment of the lancet A2, the lancing needle 1 is accommodated into the cylindrical portion 3', as shown in Fig. 15. The accommodation of the lancing needle 1 is performed by rotating the rotational member 6' relative to the cylindrical portion 3' in the circumferential direction of the housing 4 to move the lancet body 2' in the N1 direction. The detachment of the lancet A2 is performed by moving the rotational member 6' relative to the lancet holder 5 in the N2 direction. However, the detachment of the lancet A2 may be performed manually by the user.

In this embodiment again, the user can cause the lancing needle 1 to project from the lancet A2 safely without touching the lancet A2 and without performing a complicated operation. Further, the detachment of the lancet A2 after the lancing can be performed with the lancing needle 1 accommodated in the cylindrical portion 3', and the returning of the lancing needle 1 into the cylindrical portion 3' can be performed safely by the user without touching the lancet A2. Therefore, the detachment and disposal of the lancet can be performed hygienically and safely.

In the above embodiment, the lancet body is rotated with the cylindrical portion fixed to the lancet holder. However, the cylindrical portion may be rotated with the lancet body fixed to the lancet holder.

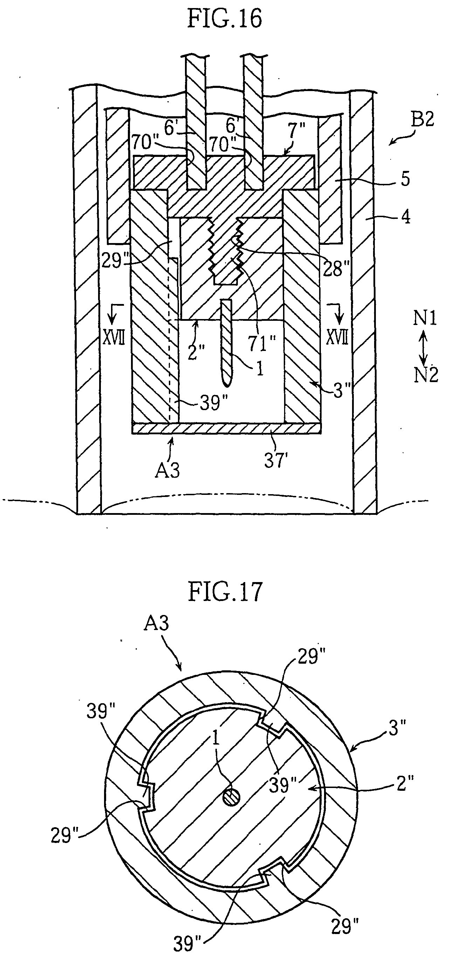

In the lancing apparatus B2, a lancet A3 as shown in Figs. 16 and 17 may be used. In Figs. 16 and 17, the elements which are identical to those of the lancing apparatus B2 or the lancet A2 described above are designated by the same reference signs.

The lancet A3 includes a lancet body 2" having a lancing needle 1, a cylindrical portion 3" for accommodating the lancing needle 1, and a rotational portion 7" which is rotatable relative to the lancet body 2" and the cylindrical portion 3" and movable relative to the lancet body 2'' in the axial direction of the lancing needle 1.

The lancet body 2" includes a female thread portion 28" having an inner surface formed with a female thread, and a guide groove 29" extending axially of the lancing needle 1. The cylindrical portion 3" includes a guide projection 39" for engagement with the guide groove 29".

The rotational portion 7" includes a recess 70" for inserting the rotational member 6' of the lancing apparatus B2, and a male thread portion 71" for engagement with the female thread portion of the lancet body 2". With such a structure, the rotational portion 7" can be rotated by using the rotational member 6'. Since the guide groove 29" of the lancet body 2" is in engagement with the guide projection 39" of the cylindrical portion 3", the lancet body 2" does not rotate relative to the cylindrical portion 3". Therefore, the rotational portion 7" rotates relative to the lancet body 2", so that the lancet body 2" and hence the lancing needle 1 advances or retreats relative to the rotational portion 7" in accordance with the rotation direction. In this way, in the lancet A3 again, the position of the lancing needle 1 can be adjusted by rotating the rotational member 6' of the lancing apparatus B2.

## Claims

1. A lancet to be attached to a lancing apparatus for moving a lancing element in a lancing direction from a standby position toward a lance position, the lancet comprising a first member including a lancing element and a second member for accommodating a tip of the lancing element, the first member and the second member being movable relative to each other;
wherein when a load greater than a predetermined value is applied in a direction to cause the first member and the second member to approach each other, the first member is brought closer to the second member so that the tip of the lancing element is capable of projecting from the lancing element, whereas when the first member is brought away from the second member, the tip of the lancing element is accommodated in the second member without projecting from the second member.

2. The lancet according to claim 1, further comprising a fixer for fixing the second member to the first member when the second member accommodates the lancing element.

3. The lancet according to claim 2, wherein the fixer comprises a pair of projections which project at the first member in a direction crossing the lancing direction and which are spaced from each other in the lancing direction, and
an engagement portion provided at the second member to be held between the paired projections.

4. The lancet according to claim 3, wherein the pair of projections comprises a first projection and a second projection which is closer to the lance position than the first projection and which projects more than the first projection.

5. The lancet according to claim 4, wherein the second projection serves as a stopper for controlling the movement of the second member by engaging with the engagement portion of the second member when the second member moves relative to the first member in the lancing direction.

6. The lancet according to claim 3, wherein at least either of the pair of projections and the engagement portion is annular.

7. The lancet according to claim 3, wherein the first member includes a hole for accommodating an end of the second member and allowing movement of the second member; and
wherein the paired projections are formed at an inner surface of the hole.

8. The lancet according to claim 7, wherein the hole has a bottom surface serving as a stopper for controlling the movement of the second member by engaging with the engagement portion of the second member when the second member moves relative to the first member in a direction opposite to the lancing direction.

9. The lancet according to claim 7, wherein the first member includes an additional hole communicating with the above-mentioned hole and extending in the lancing direction; and
wherein force is applicable to the second member in the lancing direction via the additional hole.

10. The lancet according to claim 1, wherein the second member accommodates the tip of the lancing element in a hermetically sealed state and includes a portion to be penetrated by the tip of the lancing element when the first and the second members are moved to approach each other.

11. The lancet according to claim 10, wherein the portion to be penetrated is integrally formed with the second member.

12. The lancet according to claim 11, wherein the portion to be penetrated is provided at a position retreating, in a direction opposite to the lancing direction, from an end surface of the second member on a lancing direction side.

13. The lancet according to claim 10, wherein the portion to be penetrated is provided by a sheet member attached.

14. A lancing apparatus which is used by mounting a lancet and which moves the lancet in a lancing direction from a standby position toward a lance position;
the lancet comprising a first member including a lancing element and a second member for accommodating a tip of the lancing element, the first member and the second member being movable relative to each other, wherein when a load greater than a predetermined value is applied in a direction to cause the first member and the second member to approach each other, the first member is brought closer to the second member so that the tip of the lancing element is capable of projecting from the lancing element, whereas when the first member is brought away from the second member, the tip of the lancing element is accommodated in the second member without projecting from the second member,
the lancing apparatus comprising:
a lancet holder for holding the lancet, the lancet holder being movable in the lancing direction; and
a mover which is movable relative to the lancet holder for moving the second member relative to the first member in the lancing direction, and for causing the tip of the lancing element projecting from the second member to be brought into the second member to be accommodated therein.

15. The lancing apparatus according to claim 14, wherein the mover moves in the lancing direction to engage with the second member and moves the second member relative to the first member in the lancing direction, and thereafter pushes the lancet out of the lancet holder.

16. A lancet to be attached to a lancing apparatus for moving a lancing element from a standby position toward a lance position, the lancet comprising a first member including the lancing element and a second member for accommodating a tip of the lancing element;
wherein a position of the lancing element relative to the second member is adjustable by a rotational force applied from the lancing apparatus.

17. The lancet according to claim 16, wherein the first member or the second member is rotated by the rotational force applied from the lancing apparatus.

18. The lancet according to claim 17, wherein the first member or the second member is provided with an engagement portion to which the rotational force from the lancing apparatus is applied.

19. The lancet according to claim 16, further comprising a third member which is movable relative to the first member in a movement direction of the lancing element and which is rotatable relative to the second member;
wherein the adjustment of the position of the lancing element relative to the second member is performed by rotating the third member by the rotational force applied from the lancing apparatus.

20. The lancet according to claim 19, wherein the third member is provided with an engagement portion to which the rotational force from the lancing apparatus is applied.

21. The lancet according to claim 16, wherein the lancing element is accommodated in a hermetically sealed state.

22. A lancing apparatus to which a lancet is attached and which is designed to move the lancet from a standby position toward a lance position;
wherein the lancet comprises a first member including a lancing element and a second member for accommodating a tip of the lancing element, wherein a position of the lancing element relative to the second member is adjustable by application of a rotational force;
the lancing apparatus also comprises a rotator for applying, to the lancet, the rotational force for adjusting the relative position of the lancing element.

23. The lancing apparatus according to claim 22, wherein the lancet accommodates the lancing element in a hermetically sealed state,
the rotator being capable of moving the lancing element in the lancing direction before lancing to break the hermetically sealed state, and capable of causing the lancing element to project from the second member.
